Europäisches Patentamt

European Patent Office

Office européen des brevets

(11) Veröffentlichungsnummer: **0 228 339**
**A2**

## (12) EUROPÄISCHE PATENTANMELDUNG

(21) Anmeldenummer: 86730168.1

(22) Anmeldetag: 20.10.86

(51) Int. Cl.⁴: **A 61 B 17/56**
A 61 B 17/16

(30) Priorität: 28.10.85 DE 3538654

(43) Veröffentlichungstag der Anmeldung:
08.07.87 Patentblatt 87/28

(84) Benannte Vertragsstaaten:
AT CH DE ES FR GB IT LI NL

(71) Anmelder: **MECRON MEDIZINISCHE PRODUKTE GMBH**
**Nunsdorfer Ring 23-27**
**D-1000 Berlin 48 (DE)**

(72) Erfinder: **Anapliotis, Emmanuel**
**Kiebitzweg 5**
**D-1000 Berlin 53 (DE)**

(74) Vertreter: **Christiansen, Henning, Dipl.-Ing.**
**Dietrich-Schäfer-Weg 21**
**D-1000 Berlin 41 (DE)**

(54) Bohrsystem zum Einbringen einer Endoprothese.

(57) Bohrsystem für eine Endoprothese mit einer Bohrlehre, die aufweist: einen geraden stielartigen Schaft sowie ein eine durchgehende Bohrung aufweisendes Führungselement für einen Bohrer, welches mit dem stielartigen Schaft verbunden ist, wobei die Bohrung in einem Winkel auf den Schaft zu gerichtet ist, sich die Achsen von Schaft und Bohrung im wesentlichen schneiden und das Führungselement fest mit dem zylindrischen stielartigen Schaft verbunden ist.

Fig. 1

**Beschreibung**

Bohrsystem zum Einbringen einer Endoprothese

Die Erfindung betrifft ein Bohrsystem der im Oberbegriff des Anspruchs 1 angegebenen Art sowie eine zugehörige Prothese.

Bei der Fixation von zementfreien Prothesen besteht ein Weg der Befestigung darin, daß eine sogenannte "Press-fit"-Situation geschaffen wird. Das bedeutet, daß der Femur-Markraum mit bester Präzision so angepaßt wird, daß eine Prothese der richtig gewählten Größe für das jeweilige Femur mit leichter Pressung in den vorbereiteten Markraum eingeschlagen werden kann. Das Anpassen des Markraums an verschiedene Prothesengrößen kann dabei nur durch ein mechanisches Ausräumsystem erfolgen. Das Ausräumen mit der konventionellen Raspel führt jedoch zu Achsfehlern und schafft mit großer Wahrscheinlichkeit einen im proximalen Bereich zu großen Raum, womit eine Lockerung praktisch bereits vorprogrammiert ist.

Aus der DE-A-2 356 464 ist ein Bohrsystem gemäß Oberbegriff des Anspruchs 1 bekannt. In der zugehörigen Bohrlehre ist das den Bohrer in einer auf den Stiel gerichteten Schrägstellung ausrichtende Führungselement in bezug auf den stielartigen Schaft in dessen Längsrichtung verschieblich. Diese Verschiebung dient zum vollständigen Ausfräsen des an den zylindrischen Schaft anschließenden prismatischen Bereichs der Ausnehmung zur Aufnahme des entsprechenden Teils der Prothese. Dabei wird der Fräser in seiner auf den Schaft gerichteten Schrägstellung bei in die zuvor erzeugte Bohrung eingesetztem Schaft entlang dieses Schaftes an den Knochen herangeführt bis er beginnt, die vorhandene Bohrung um ein dreieckiges Prisma zu erweitern. Bei weiterem Voranschieben des in Schrägstellung geführten Fräsers wird der prismatische Bereich vollständig ausgeräumt.

Nachteilig ist dabei, daß die Ausfräsung vorwiegend entlang der Seitenlinie des Fräsers erfolgt, so daß eine den stielartigen Schaft biegende bzw. aus seiner Führung im Knochen herausbrechende Kraftkomponente (in Form eines Beigemomentes) auftritt. Weiterhin muß der gesamte prismatische Bereich durch Fräsen entfernt werden, was im Operationsverlauf einen langwierigen Vorgang darstellt.

Der im kennzeichnenden Teil des Anspruchs 1 angegebenen Erfindung liegt die Aufgabe zugrunde, ein Bohrsystem der eingangs genannten Gattung, welches es ermöglicht, bei kleinerem Kraftaufwand den Fräser absolut geradlinig zu führen, so daß ein Ausbrechen oder Verlaufen des Fräsers nicht zu befürchten ist und somit der Markraum mit großer Präzision an die Abmessungen einer Geradschaftprothese angepaßt werden kann.

Der Erfindung liegt die Erkenntnis zugrunde, daß es wesentlich einfacher ist, den dreieckigen prismatischen Teil durch eine schräg eingebrachte, auf den Schaft gerichtete Bohrung in seiner Begrenzung durch eine in axialer Richtung vorangetriebene Bohrung zu definieren und den verbleibenden (kleinen) dreieckigen Restbereich durch einige Schläge mit einem Meißel herauszubrechen. Dieser Restbereich wird durch Spongiosa gebildet, welche nur eine geringe Festigkeit hat und bei den kleinen auftretenden Restbereichsgrößen ohne weiteres entfernbar ist. Ein Ausbrechen des stielartigen Schaftes aus seiner zylindrischen Vorbohrung heraus im Bereich der Spongiosa hätte dagegen von vorn herein eine Lockerung des Sitzes der Prothese zur Folge, was unbedingt vermieden werden muß.

Der von der Geradschaftprothese eingenommene Hohlraum wird durch eine erste parallel zum Knochen verlaufende gerade Bohrung und eine zweite, schräg auf diese zu gerichtete, konvergierende Bohrung erzeugt, wobei der zwischen den beiden konvergierenden Bohrungen gelegene Bereich von Knochensubstanz mit dem Einbringen der Bohrungen ebenfalls im wesentlichen seinen Halt verliert.

Dabei wird der distale Markkanal mit einem geraden Bohrer oder Fräser vorgebohrt und der proximale Raum der Prothese mit einem weiteren Bohrer mit Hilfe der Bohrlehre ausgeräumt. Dieses System bietet weitgehende Sicherheit gegen zu weitgehendes Ausräumen und ermöglicht einen gute Pressfit-Sitz für eine zementfrei implantierte Prothese. Außerdem hat das System den Vorteil, daß die Resektionsebene mit Hilfe der Bohrlehre nach dem Einbringen der Bohrung noch korrigiert werden kann und somit ein exakter Kragenaufsitz erzielt wird.

Zusätzlich kann der Bohrer noch mit einer durchgehenden Bohrung versehen sein, um beim Bohren entlang eines zuvor eingebrachten Führungsspießes geführt zu werden, so daß die Sicherheit hinsichtlich einer Perforation des Femurs zusätzlich erhöht ist. Wichtig ist, daß die Operationszeit durch die Anwendung des erfindungsgemäßen Systems insgesamt erheblich verkürzt ist.

Das System bietet somit insbesondere die folgenden Vorteile:
Durch Schaffung einer Pressfit-Situation ist das paßgenaue Implantieren und damit die primäre Fixation einer zementfreien Prothese gesichert.
Das Bohren ist mit dem Führungsspieß genau und präzise möglich.

Die Operationstechnik beim Femur erfolgt in Ablauf nacheinander mit den folgenden Maßnahmen:

1. Anbohren am hohen Trochanter mit dem Trocar.

2. Einführen eines Führungsspießes in den Markraum

3. Bohren beginnend mit dem kleinsten Bohrer über dem Führungsspieß bis in die Tiefe der Prothesenschaftlänge, ggf. unter Wandlerkontrolle, anschließend Bohren mit zunehmendem Querschnitt bis zum Durchmesser des Schaftes der Bohrlehre.

4. Resektion des Hüftkopfes.

5. Einführen der Bohrlehre

6. Korrektur der Hüftkopfosteotomie.

7. Bohren der zweiten Bohrung längs des Adamschen Bogens bis zum Anschlag der Bohrlehre.

8. Ausräumen der Restspongiosa mit einem flachen Meißel, falls erforderlich.

9. Paßgenaues Einschlagen der Prothese.

10. Nachschlagen mit dem Impaktor.

Dadurch, daß der rund ausgebildete Schaft und die Bohrung von deren Führungselement im Durchmesser derart aufeinander abgestimmt sind, daß der Schaft von einer Bohrung im Knochen im wesentlichen spielfrei geführt wird, die mittels eines Bohrers erzeugt ist, der seinerseits von der Bohrung im wesentlichen spielfrei geführt wird und insbesondere der Durchmesser der Bohrung gleich oder geringfügig größer ist als der Durchmesser des Schafts, lassen sich beide Bohrungen mit demselben Bohrer oder Fräser erzeugen. Da beide Bohrungen in axialer Richtung vorangetrieben werden, ist kein Fräser erforderlich, der in radialer Richtung schneidet.

Wenn gemäß einer vorteilhaften Weiterbildung die Bohrlehre in demjenigen Bereich, in dem ein sich durch die Bohrung hindurch erstreckender Bohrer auf diesen auftrifft, eine Ausnehmung aufweist, welche den Weg des Bohrers in Richtung auf die Prothese durch eine sich quer erstreckende Oberfläche begrenzt und der Bohrer insbesondere bis zu seinem maximalen Querschnitt in diese Ausnehmung eintritt, ist der dreieckförmige prismatische Bereich an seinen Seitenkanten vollkommen frei, so daß nur noch die Deckflächen dieses Bereiches später durch Meißeln gelöst werden müssen.

Bei einer anderen bevorzugten Ausführung weist das die Bohrung enthaltende Führungselement der Bohrlehre an derjenigen Seite, an die Achsen von Schaft und Bohrung konvergieren, eine Anschlagfläche auf, deren Flächenvektor im wesentlichen in die von den Mittelachsen des Schaftes und der Bohrung aufgespannte Fläche fällt. Diese Fläche liegt auf dem reduzierten Femur auf und entspricht dem "Kragen" einer eventuell später in die erzeugte Ausnehmung einzusetzenden Prothese. Es ist ersichtlich, daß der Schaft in seinem in die zuvor erzeugte Bohrung einzusetzenden Bereich zuylindrisch oder in einen zylindrischen Körper einführbar ausgebildet ist.

Vorteilhafte Weiterbildungen der Erfindung sind in den übrigen Unteransprüchen gekennzeichnet bzw. werden nachstehend zusammen mit der Beschreibung der bevorzugten Ausführung der Erfindung anhand der Zeichnung näher dargestellt. Es zeigen

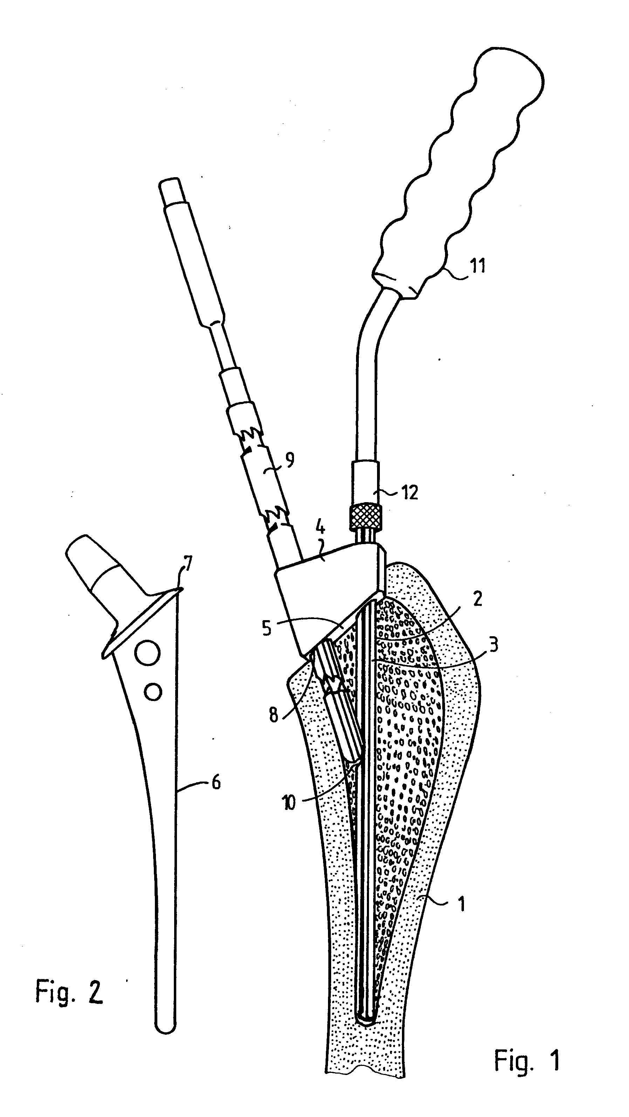

Figur 1 ein Ausführungsbeispiel des erfindungsgemäßen Bohrsystems, bestehend aus Bohrschablone und Bohrer, sowie

Figur 2 eine diesem Bohrsystem angepaßte Prothese.

In den Femur 1 ist zunächst eine Bohrung 2 eingebracht worden, in die der Schaft 3 der Bohrlehre als Teil des erfindungsgemäßen Bohrsystems eingeführt wird. Dabei ist gleichzeitig eine Kontrolle bezüglich der Tiefe der Bohrung gegeben, da bei ordnungsgemäßem Sitz der Prothese ein Führungselement 4, dessen Unterseite 5 eine Neigung aufweist, die derjenigen des Kragens der in die Ausnehmung einzusetzenden Prothese entspricht, auf dem Knochen aufsitzen muß. In der Figur ist neben der Bohrlehre die Kontur einer Geradschaftprothese 6 mit Kragen 7 wiedergegeben, wobei ohne weiteres erkennbar ist, daß der mit dem erfindungsgemäßen Bohrsystem ausgeräumte Knocheninnenraum weitgehend der Kontur der Prothese entspricht. Die durch die Unterseite 5 der Bohrlehre gebildete (Anschlag-)fläche entspricht der Neigung der Unterseite des Kragens der zugehörigen entsprechend ausgerichteten Prothese, so daß insoweit der Sitz der Bohrlehre eine gute Beurteilung des späteren Porthesensitzes erlaubt und gegebenenfalls rechtzeitig eine Nachbearbeitung des Knochenmaterials vorgenommen werden kann.

Das Führungselement 4 weist eine Bohrung 8 auf, die an den Querschnitt eines Bohrers 9 angepaßt ist. Diese Bohrung ist so angeordnet, daß der Bohrer 9 auf den Schaft 3 hin gerichtet ist und diesen im Bereich einer Ausnehmung 10 trifft, die derart gestaltet ist, daß sie den Weg des Bohrers in Richtung auf den Schaft begrenzt. Der Durchmesser der Bohrung 8 entspricht dem Durchmesser des Schafts 3 derart, daß der Bohrer, welcher die Ausnehmung für die Aufnahme des Schafts geschaffen hat, seinerseits durch die Bohrung richtungsmäßig geführt wird.

Diese Ausnehmung 10 weist eine Kante auf, welche die Fortsetzung des nahe dem Schaft gelegenen Teils bildet, durchquert den Schaft 1 aber nur teilweise und geht in eine Fläche über, die quer zur Achse der Fortsetzung der Bohrung gerichtet ist und einen Anschlag für den Bohrer 9 bildet. Benachbart derjenigen Seite des Führungselementes 4 gelegenen Teil, an der die Bohrung 8 und der Schaft 3 divergieren, ist ein Griff 11 nach einer Abwinklung vorgesehen, welche es ermöglicht, einerseits die Bohrlehre sicher beim Einsetzen und Herausnehmen zu führen und andererseits diese auch gegen ein unbeabsichtigtes Verdrehen zu sichern, bevor der in die Bohrung 8 eingesetzte Bohrer 9 gefaßt hat. Durch die Abbiegung des Griffs ist der Bereich der Bohrung 8 zur Handhabung frei.

Der Handgriff 11 ist mit dem Schaft mittels einer Schnellkupplung verbunden, die eine mit einer Rändelung versehene Überwurf-Schraubhülse 12 aufweist. Innerhalb der Schraubhülse befindet sich eine Steckverbindung, welche die trennbaren Teile drehmomentschlüssig verbindet. Auf diese Weise läßt die Verbindung (die in der Zeichnung nicht näher dargestellt ist), die Übertragung von Kräften und Momenten in allen Richtungen zu.

Wenn die beiden Bohrungen in den Femurbereich eingebracht sind, besteht die Sicherheit, daß der Prothesenschaft einer Press-Fit-Prothese genau und ausgerichtet paßt.

Knochenmaterial, welches eventuell zwischen den beiden Bohrungen stehenbleibt, braucht mit dem Meißel nur grob beseitigt zu werden, da dieses Material nicht die Führung der Prothese beeintächtigt, sondern zusätzlich deren Preßsitz verbessert.

Durch das Führungselement, welches bezüglich seiner unteren Fläche als Anschlag in seiner Funktion dem Kragen der künftigen Prothese entspricht, läßt sich auch ohne weiteres visuell die richtige Ausrichtung der Bohrlehre zur Niederbringung der Prothese beurteilen bzw. gegebenenfalls die Notwendigkeit einer verbesserten Anpassung durch zusätzliches Abtragen von Knochenmaterial.

An das erfindungsgemäße Bohrsystem, bestehend aus der beschriebenen Bohrlehre und einem daran querschnittsmäßig angepaßten Bohrer bzw. Fräser ist eine entsprechende Geradschaftprothese, wie sie durch die Kontur 6 repräsentiert ist. Der Schaft geht vom proximalen zum distalen Ende hin vom ovalen in den runden Querschnitt über. Die Außenkante verläuft bis zum Kragen 7 gerade, während die Innenkante in kontinuierlicher Krümmung von der Richtung des geraden Schaftes der Bohrlehre in die Richtung der Bohrung von deren Führungselement übergeht. Die Unterseite des Kragens 7 weist dementsprechend eine flächenmäßige Ausrichtung auf, die derjenigen der Unterseite des Führungselements der Bohrlehre entspricht.

Der Schaft der Prothese 6 wird somit in seiner Außenkontur im wesentlichen bestimmt durch die Überlagerung des Schaftes 3 der Bohrlehre und eines in die Bohrung 8 des Führungselements 4 eingesetzten runden Bohrers 9, der bis an den Schaft heranreicht, sowie desjenigen Volumens, das von den Flächen eingeschlossen wird, die Schaft und Bohrer tangieren und zwischen diesen beiden gelegen ist. Die Prothese weist dabei einen Kragen 7 auf, dessen Unterfläche bezüglich der räumlichen Richtung der Fläche 5 an dem Führungselement 4 entspricht, deren Flächenvektor im wesentlichen in die von den Achsen des Schaftes und der Bohrung aufgespannte Fläche fällt.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Bohrsystem zum Einbringen einer Endoprothese, mit einer Bohrlehre, die aufweist: einen zylindrischen stielartigen Schaft (3) sowie ein eine durchgehende Bohrung (8) aufweisendes Führungselement (4) mit einer zylindrischen Ausnehmung für einen Fräser (9), welches mit dem Schaft verbunden ist, wobei die Ausnehmung in einem Winkel auf den Schaft zu gerichtet ist und sich die Achsen von Schaft und Bohrung im wesentlichen schneiden, **dadurch gekennzeichnet,** daß das Führungselement (4) fest mit dem zylindrischen stielartigen Schaft (3) verbunden ist.

2. Bohrsystem nach Anspruch 1, **dadurch gekennzeichnet,** daß der rund ausgebildete Schaft (3) und die Bohrung (8) von deren Führungselement (4) im Durchmesser derart aufeinander abgestimmt sind, daß der Schaft von einer Bohrung im Knochen im wesentlichen spielfrei geführt wird, die mittels eines Bohrers (9) erzeugt ist, der seinerseits von der Bohrung im wesentlichen spielfrei geführt wird.

3. Bohrsystem nach Anspruch 1, **dadurch gekennzeichnet,** daß der Durchmesser der Bohrung (8) gleich oder geringfügig größer ist als der Durchmesser des Schafts (3).

4. Bohrsystem nach einem der vorangehenden Ansprüche, **dadurch gekennzeichnet,** daß der Schaft (3) der Bohrlehre in demjenigen Bereich, in dem ein sich durch die Bohrung hindurch erstreckender Bohrer (9) auf diesen auftrifft, eine Ausnehmung (10) vorgesehen ist, welche den Weg des Bohrers in Richtung auf die Prothese durch eine sich quer erstreckende Oberfläche begrenzt.

5. Bohrsystem nach einem der vorangehenden Ansprüche, **dadurch gekennzeichnet,** daß das die Bohrung (8) aufweisende Führungselement (4) der Bohrlehre an derjenigen Seite, an die Achsen von Schaft und Bohrung konvergieren, eine Anschlagfläche (5) aufweist, deren Flächenvektor im wesentlichen in die von den Mittelachsen des Schaftes und der Bohrung aufgespannte Fläche fällt.

6. Bohrsystem nach einem der vorangehenden Ansprüche, **dadurch gekennzeichnet,** daß der Schaft (3) der Bohrlehre an demjenigen Ende, an dem die Achsen von Schaft und Bohrung divergieren, einen Haltegriff (11) aufweist.

7. Bohrsystem nach Anspruch 7, **dadurch gekennzeichnet,** daß das Griffende abgebogen ist, wobei die Abbiegung sich von der Achse der Bohrung zunehmend entfernt.

8. Bohrsystem nach Anspruch 7, **dadurch gekennzeichnet,** daß der Haltegriff (11) demontierbar ausgebildet ist.

9. Bohrsystem nach Anspruch 7, **dadurch gekennzeichnet,** daß der Haltegriff (11) mittels einer eine Überwurfschraubhülse (12) und einer momentschlüssigen Steckverbindung aufweisenden Schnellkupplung mit dem Schaft verbunden ist.

10. Bohrsystem nach einem der vorangehenden Ansprüche, **dadurch gekennzeichnet,** daß der Fräser oder Bohrer (9) zum Ausräumen von Knochenmaterial querschnittsmäßig an die Bohrung des Führungselements (4) der Bohrlehre angepaßt ist.

Fig. 2

Fig. 1